# EUROPEAN PATENT SPECIFICATION

(11) **EP 2 370 452 B1**
(45) Date of publication and mention of the grant of the patent: **05.04.2017**
(21) Application number: 09796343.3
(22) Date of filing: 03.12.2009
(51) Int. Cl.: C07K 1/14, G01N 33/483

(54) **PROCESS FOR EXTRACTING PROTEINS FROM FFPE BIOLOGICAL TISSUE SAMPLES**
VERFAHREN ZUR EXTRAKTION VON PROTEINEN AUS BIOLOGISCHEN FFPE-GEWEBEPROBEN
PROCÉDÉ D'EXTRACTION DE PROTÉINES À PARTIR D'ÉCHANTILLONS TISSULAIRES BIOLOGIQUES FFPE

(30) Priority: 04.12.2008 IT MI20082148
(43) Date of publication of application: 05.10.2011
(73) Proprietor: Porto Conte Ricerche S.r.l., 07041 Alghero (IT)
(72) Inventor: ADDIS, Maria, Filippa, I-07100 Sassari (IT); UZZAU, Sergio, I-07100 Sassari (IT); TANCA, Alessandro, I-07100 Sassari (IT)
(74) Representative: Gervasi, Gemma
(86) International application number: PCT/EP2009/066317
(87) International publication number: WO 2010/063796

(56) References cited:
- DE-A1-102005 023 011
- US-A1- 2002 197 224
- US-A1- 2009 069 549
- ESPINA VIRGINIA ET AL: "Protein microarrays: molecular profiling technologies for clinical specimens." PROTEOMICS NOV 2003, vol. 3, no. 11, November 2003 (2003-11), pages 2091-2100, XP002530016 ISSN: 1615-9853
- NIRMALAN NIROSHINI J ET AL: "Development and validation of a novel protein extraction methodology for quantitation of protein expression in formalin-fixed paraffin-embedded tissues using western blotting." THE JOURNAL OF PATHOLOGY MAR 2009, vol. 217, no. 4, 1 December 2008 (2008-12-01), pages 497-506, XP002530017 ISSN: 1096-9896
- SHI SHAN-RONG ET AL: "Protein extraction from formalin-fixed, paraffin-embedded tissue sections: quality evaluation by mass spectrometry." THE JOURNAL OF HISTOCHEMISTRY AND CYTOCHEMISTRY : OFFICIAL JOURNAL OF THE HISTOCHEMISTRY SOCIETY JUN 2006, vol. 54, no. 6, June 2006 (2006-06), pages 739-743, XP002530018 ISSN: 0022-1554
- BELIEF V ET AL: "Proteomic analysis of RCL2 paraffin-embedded tissues." JOURNAL OF CELLULAR AND MOLECULAR MEDICINE OCT 2008, vol. 12, no. 5B, October 2008 (2008-10), pages 2027-2036, XP002530019 ISSN: 1582-1838
- ANONYMOUS: 'SuperScript (TM) III Reverse Transcriptase', [Online] 07 December 2004, XP055217463 Retrieved from the Internet: <URL:https://tools.thermofisher.com/content /sfs/manuals/superscriptIII_man.pdf> [retrieved on 2015-10-01]
- 'Biochemicals, reagents & kits', 01 January 2006, SIGMA-ALDRICH deel 'Renaturation Basic Kit for Proteins, 96827', page 2067, XP055217524
- NOVAGEN ET AL: 'Protein Refolding Kit' INTERNET CITATION, [Online] December 1998, XP002426307 Retrieved from the Internet: <URL:http://wolfson.huji.ac.il/purification /PDF/Protein_Refolding/NOVAGEN_Protein_Refo lding_kit.pdf> [retrieved on 2007-03-23]
- MARIA FILIPPA ADDIS ET AL: 'Generation of high-quality protein extracts from formalin-fixed, paraffin-embedded tissues' PROTEOMICS vol. 9, no. 15, 01 August 2009, pages 3815 - 3823, XP055113247 DOI: 10.1002/pmic.200800971 ISSN: 1615-9853
- MARIA FILIPPA ADDIS ET AL: '2-D PAGE and MS analysis of proteins from formalin-fixed, paraffin-embedded tissues' PROTEOMICS vol. 9, no. 18, 28 August 2009, pages 4329 - 4339, XP055113248 DOI: 10.1002/pmic.200900010 ISSN: 1615-9853
- ALESSANDRO TANCA ET AL: 'Application of 2-D DIGE to formalin-fixed, paraffin-embedded tissues' PROTEOMICS vol. 11, no. 5, 31 March 2011, pages 1005 - 1011, XP055113249 DOI: 10.1002/pmic.201000353 ISSN: 1615-9853
- ALESSANDRO TANCA ET AL: 'Proteomic analysis of formalin-fixed, paraffin-embedded lung neuroendocrine tumor samples from hospital archives' JOURNAL OF PROTEOMICS vol. 74, no. 3, 01 March 2011, pages 359 - 370, XP055113250 DOI: 10.1016/j.jprot.2010.12.001 ISSN: 1874-3919
- ALESSANDRO TANCA ET AL: "Impact of fixation time on GeLC MS/MS proteomic profiling of formalin-fixed, paraffin-embedded tissues", JOURNAL OF PROTEOMICS, ELSEVIER, AMSTERDAM, NL, vol. 74, no. 7, 14 March 2011 (2011-03-14) , pages 1015-1021, XP028387672, ISSN: 1874-3919, DOI: 10.1016/J.JPROT.2011.03.015 [retrieved on 2011-03-23]
- ANONYMOUS: 'Buffers and Standard Solutions', [Online] 19 December 2006, XP055113362 Retrieved from the Internet: <URL:http://ivaan.com/protocols/151.html> [retrieved on 2014-04-10]
- B. ILKOVSKI: 'Evidence for a dominant-negative effect in ACTA1 nemaline myopathy caused by abnormal folding, aggregation and altered polymerization of mutant actin isoforms' HUMAN MOLECULAR GENETICS vol. 13, no. 16, 15 June 2004, pages 1727 - 1743, XP055113520 DOI: 10.1093/hmg/ddh185
- Xiaogang Jiang ET AL: "Development of Efficient Protein Extraction Methods for Shotgun Proteome Analysis of Formalin-Fixed Tissues", Journal of Proteome Research, vol. 6, no. 3, 1 March 2007 (2007-03-01), pages 1038-1047, XP055113277, ISSN: 1535-3893, DOI: 10.1021/pr0605318

## Description

### FIELD OF THE INVENTION

The present invention relates to the field of protein extraction from biological tissue samples.

### STATE OF THE ART

Archives of tissue fixed in formalin and embedded in paraffin (FFPE, an acronym of Formalin-Fixed, Paraffin-Embedded) contain a large amount of information on the proteins involved in various aspects of many pathological conditions. These archives are the result of immunohistochemical diagnostic procedures undertaken in the anatomy and pathology laboratories of hospitals throughout the world. Were they to be accessible, the protein molecules of FFPE tissue would be an invaluable resource in terms of biomarker research, especially if considering the significance of retrospective information relating to the diagnosis, prognosis and outcome of a specific pathology.

Within the last few years much effort has been aimed at making access to this "hidden treasure" possible. Work carried out in this field has led to the development of various strategies aimed at extracting the proteins from FFPE tissue samples.

However, protein extraction from FFPE tissue is hindered by the chemistry of fixation with formaldehyde and, despite some methods having given satisfactory results, they often show limited potential for application to analytical techniques following extraction. Indeed, poor extraction yields and poor extracted protein quality ensue from these methods; furthermore, the condition in which they are obtained renders them incompatible with the most widely used techniques of proteomic analysis.

Document WO2006/122898 A1 described a hot extraction method for protein from a FFPE biological sample in which the biological sample is incubated at a temperature suitable for protein release in the absence of active proteolytic compounds, in a buffer containing a detergent. According to that described in the examples, said method was implemented by using a buffer at pH 6.8, optionally in the presence of a reducing agent dithiothreitol (DTT) at a 1 mM concentration. For the protein extracts thus obtained, an expression analysis could be applied based on reverse-phase protein microarrays.

However, the extraction methods obtained so far have not resulted in an extracted FFPE protein sample of a quality that enables analyses to be applied such as polyacrylamide gel electrophoresis (SDS-PAGE) separation, differential proteomic or mass spectroscopy (MS) procedures based on in-gel digestion of SDS-PAGE separated protein bands and especially 2D-PAGE combined with MALDI-MS, this latter currently being the most widespread and practical analysis technique in the field of differential proteomics.

In the very recent document "Proteomic analysis of RCL2 paraffin-embedded tissues", V. Bellet et al., J.Cell. Mol. Med., Vol.12, No.58, October 2008, pages 2027-2036, FFPE samples were deemed inappropriate for nucleic acid and protein studies and RCL2 paraffin-embedded tissues are proposed.

The object of the present invention is hence to provide a reliable and versatile method for extracting proteins from FFPE samples, simultaneously enabling high quality and high yield extracts to be obtained, which are compatible with most of the proteomic methods such as analysis by SDS-PAGE, western immunoblotting and protein arrays. In particular the present invention provides a method that enables protein extracts to be obtained which are suitable for a subsequent combined application of 2D-PAGE and MALDI-MS.

### SUMMARY OF THE INVENTION

The aforesaid purpose and the advantages which will be described were achieved by means of a process for extracting proteins from FFPE tissue samples comprising the following steps:
i) deparaffinizing the FFPE sample,
ii) adding to the deparaffinized FFPE sample an extraction buffer comprising:
   A) a buffer solution at a pH higher than or equal to 8; and
   B) a reducing agent at a concentration higher than or equal to 100 mM;
iii) incubating the FFPE sample in the extraction buffer at a temperature in the range from 55ºC to 100ºC,
in which said reducing agent is a compound chosen from the group consisting of dithiothreitol (DTT), dithioerythritol (DTE), tris(2-carboxyethyl)phosphine (TCEP), mercaptoethanol (MEA).

Another aspect of the invention concerns the use of a kit for extracting proteins from FFPE samples according to the process of the invention, said kit as a combined product for simultaneous, separate or sequential use, said kit comprising:
a) a buffer solution at a pH higher than or equal to 8;
b) a reducing agent at a concentration higher than or equal to 100 mM
in which said reducing agent is a compound chosen from the group consisting of dithiothreitol (DTT), dithioerythritol (DTE), tris(2-carboxyethyl)phosphine (TCEP), mercaptoethanol (MEA).

The process of the present invention has unexpectedly provided protein extracts from FFPE tissue samples with higher yields and quality than those obtainable using known methods, and comparable to those obtainable from the corresponding fresh-frozen samples.

The present invention has the advantage of allowing protein extracts to be obtained which can be used directly in the most widely used proteomic techniques without further processing. In this respect, SDS-PAGE, western immunoblotting and protein array analysis can be applied to the obtained extracts, and those proteins separated by SDS-PAGE and 2D-PAGE can be subsequently analyzed using mass spectrometry techniques such as MALDI-TOF-MS (Matrix-Assisted Laser Desorption/Ionization - Time-of-Flight Mass Spectrometry) and nanoHPLC-nano-ESI-QTOF-MS/MS (nano-High Pressure Liquid Chromatography-nano-ElectroSpray Ionization-Quadrupole Time of Flight Tandem Mass Spectrometry).

### DESCRIPTION OF THE FIGURES

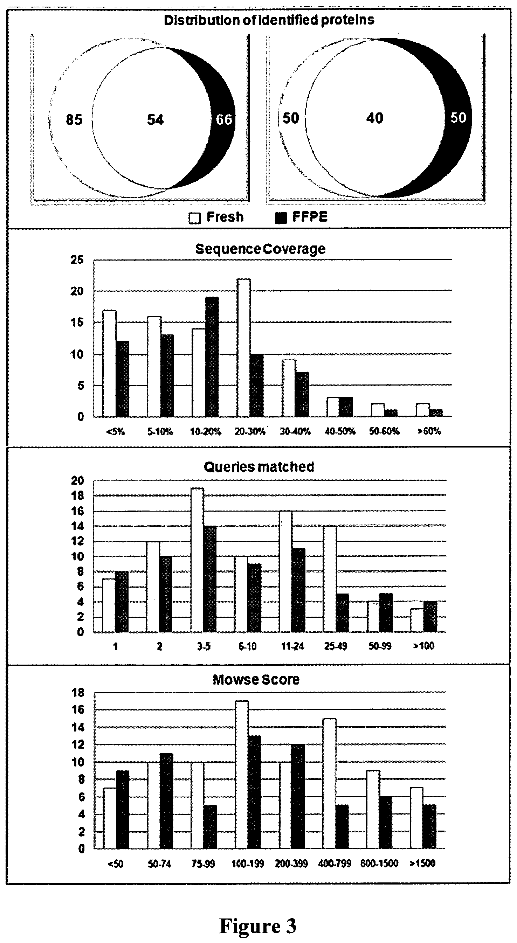

The invention will now be described in detail with reference to some figures wherein:

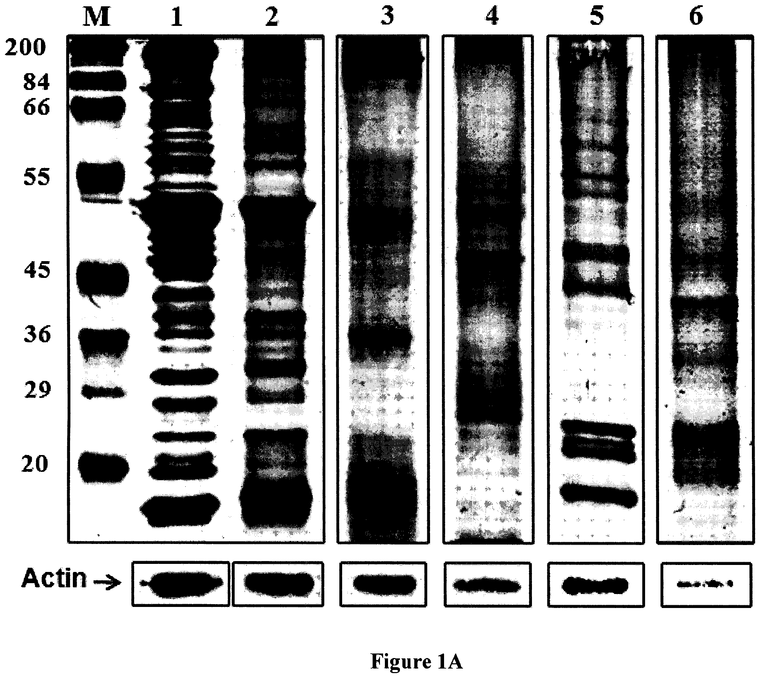

- Figure 1A illustrates the polyacrylamide gel electrophoresis (SDS-PAGE) results of proteins extracted, according to the method of the present invention, from fresh-frozen tissue and from formalin-fixed paraffin-embedded (FFPE) tissue. In the figure, M indicates the molecular weight marker; 1: fresh tissue of skeletal muscle; 2: FFPE tissue of skeletal muscle; 3: FFPE tissue of hypertrophic human thyroid; 4: healthy human liver FFPE; 5: pulmonary carcinoid FFPE; 6: pulmonary carcinoma FFPE. Reactivity with anti-actin antibodies is shown below the corresponding total protein profile. The proteins obtained with the method of the invention in example 1 were quantified using the Bradford method, about 10 micrograms of proteins in total being loaded for the SDS-PAGE and 1 microgram of total proteins for the western blotting, respectively;
- Figure 1B is a graph showing a comparison between the relative density profiles of SDS-PAGE separated proteins from fresh muscle tissue (dark grey) and from FFPE muscle tissue (light grey);
- Figure 2A shows the results of the observed reactivity, with western blot and protein arrays, of the proteins extracted according to the method of the present invention from the fresh and FFPE tissue samples obtained in example 1. In the figure, M represents the molecular weight marker;
- Figure 2B shows the results of the reactivity of the proteins GAPDH (glyceraldehyde 3-phosphate dehydrogenase), tropomyosin, vinculin, desmin and myosin in the ELISA test. The mean and standard deviation values are given for optical density at 650 nm for three independent experimental replicates;
- The top of Figure 3 shows Venn diagrams which illustrate the distribution of identified proteins from extracts of fresh tissue (white) and FFPE tissue (dark grey) obtained by the method of the present invention. In the left hand square, 64% of proteins identified in fresh tissue are also identified in FFPE tissue, while 85% of proteins identified in FFPE are also identified in fresh tissue. In the right hand square for the 50 most represented proteins, 80% are identified in both fresh and FFPE tissue. Also shown in figure 3 are three histograms representing the statistics relating to the identification of proteins from fresh tissue (light grey) and FFPE tissue (dark grey);

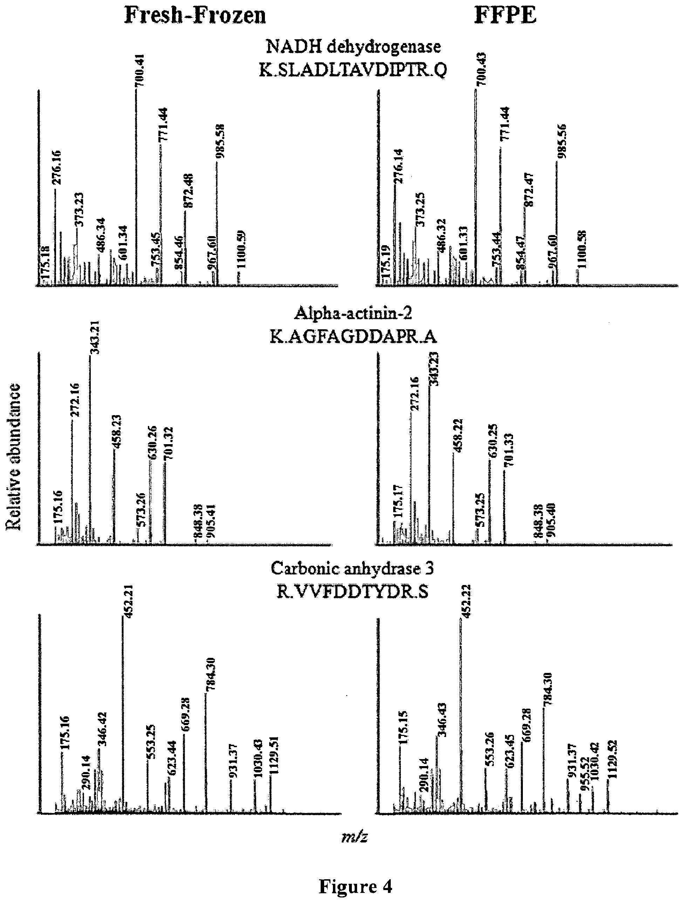

- Figure 4 shows a comparison of the mass spectra representative of peptides identified in extracts of fresh and FFPE ovine skeletal muscle tissue using the method of the present invention;

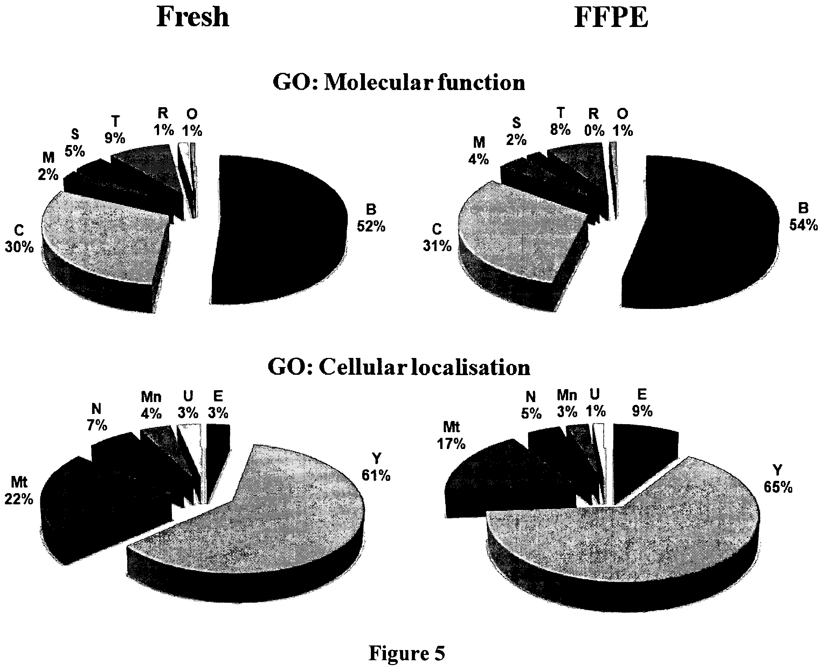

- Figure 5 shows a comparison of the distribution of proteins identified from fresh tissue (left) and FFPE tissue (right) extracts, obtained by the method of the present invention, according to the gene classification criteria (Gene Ontology, GO). The top diagram indicates the following functional protein classes: B, binding; C, catalytic; M, motor; S, structural; T, transport; R, transcription/regulation; O: other. The bottom diagram indicates the following subcellular localisations: E, extracellular; Y, cytoplasmic; Mt, mitochondrial; N, nuclear; Mn, membrane; U, other or not defined;

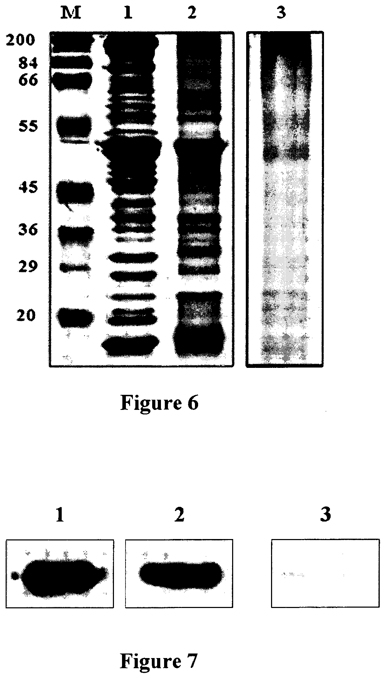

- Figure 6 illustrates the result obtained after the following: 1) extraction from fresh skeletal muscle tissue; 2) extraction from FFPE ovine skeletal muscle tissue using the method of the invention; 3) extraction from FFPE ovine skeletal muscle tissue using the method described in patent WO2006/122898 A1;
- Figure 7 illustrates a result representative of that obtained after electrophoresis and western immunoblotting with anti-actin antibodies. 1) Actin signal obtained following protein extraction from fresh skeletal muscle tissue; 2) actin signal obtained following protein extraction from five 10µm slices of FFPE skeletal muscle using the present process; 3) actin signal obtained following protein extraction from five 10µm slices of FFPE skeletal muscle using the method of patent application WO2006/122898 A1;

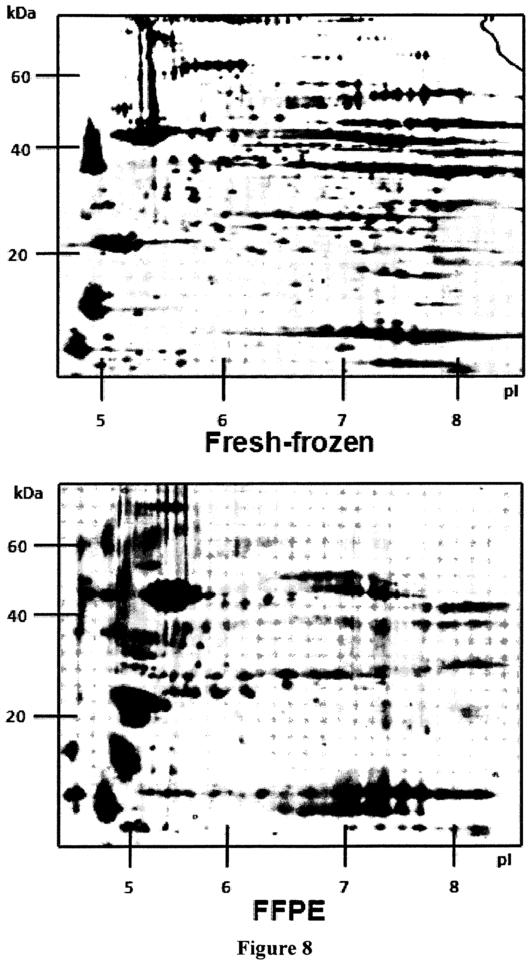

- Figure 8 shows the extract obtained from fresh and FFPE skeletal muscle with the process of the invention analyzed by two-dimensional electrophoresis;

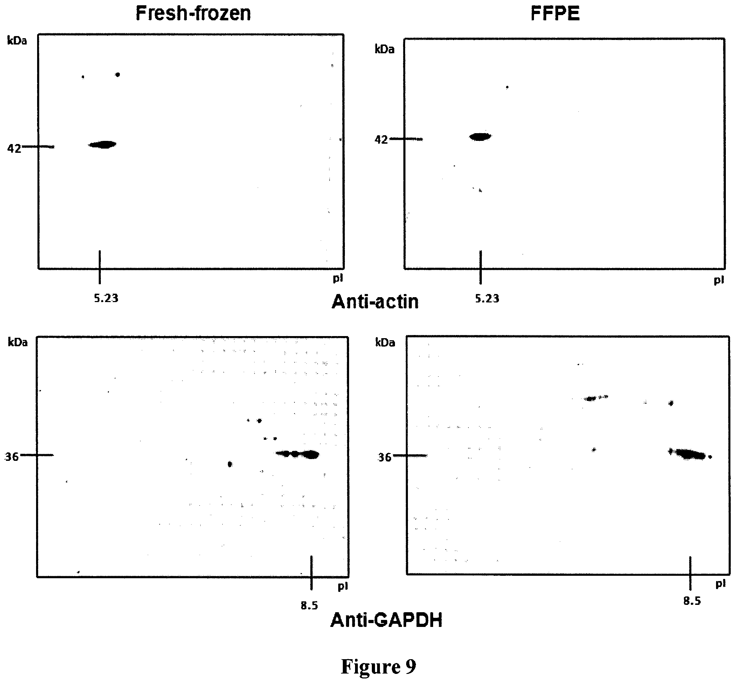

- Figure 9 shows the extract obtained from fresh and FFPE skeletal muscle with the process of the invention analyzed by two-dimensional western immunoblotting;

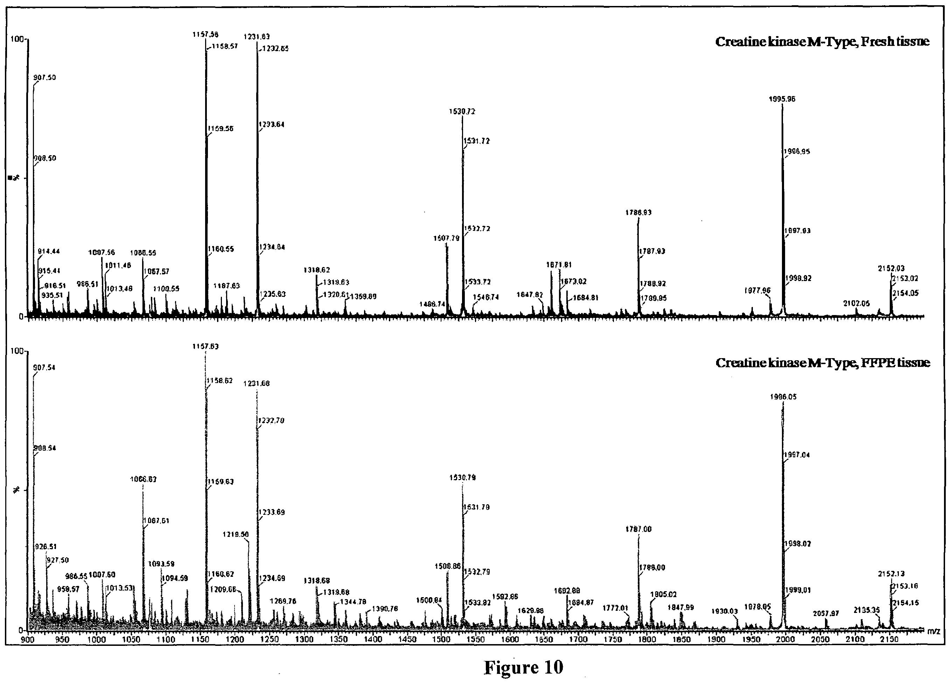

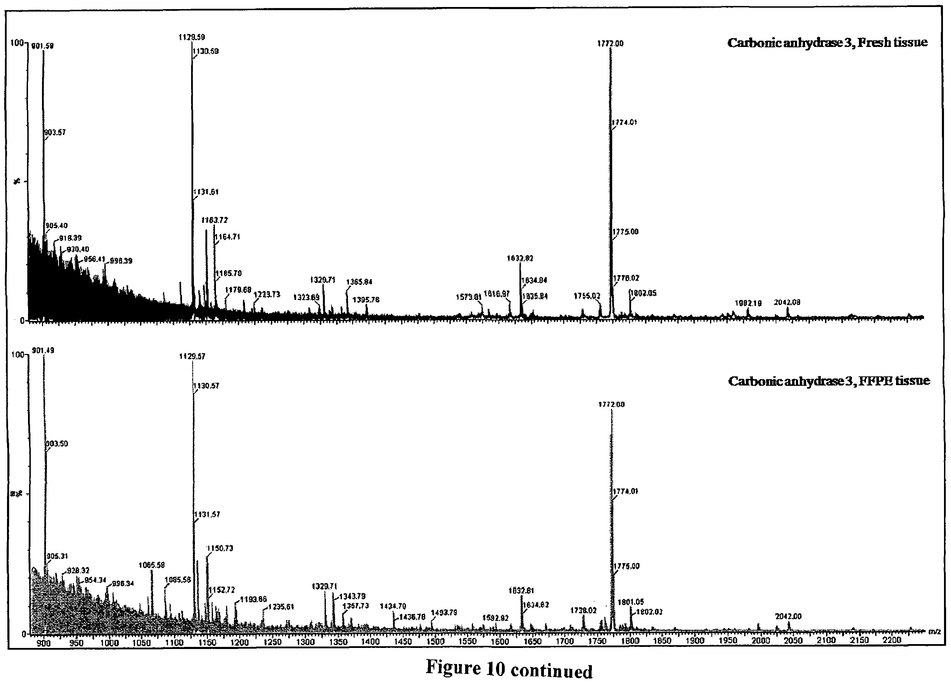

- Figure 10 shows the extract obtained from fresh and FFPE skeletal muscle with the process of the invention analyzed by mass spectrometry.

### DETAILED DESCRIPTION OF THE INVENTION

The present invention relates to a process for extracting proteins from FFPE tissue samples comprising the step ii) of adding to the FFPE sample an extraction buffer comprising:
A) a buffer solution at a pH higher than or equal to 8; and
B) a reducing agent at a concentration higher than or equal to 100 mM;
and the step iii) of incubating the FFPE sample in the extraction buffer at a temperature in the range from 55°C to 100°C,
in which said reducing agent is a compound chosen from the group consisting of dithiothreitol (DTT), dithioerythritol (DTE), tris(2-carboxyethyl)phosphine (TCEP), mercaptoethanol (MEA).

The process of the invention therefore comprises of a step ii) of adding the extraction buffer to the FFPE sample. Said extraction buffer comprises a reducing agent chosen from the group consisting of dithiothreitol (DTT), dithioerythritol (DTE), tris(2-carboxyethyl)phosphine (TCEP) and mercaptoethanol (MEA). More preferably said reducing agent is the compound dithiothreitol (DTT).

The reducing agent of the present invention is in an amount, in the extraction buffer, higher than or equal to 100 mM. Preferably said reducing agent is present in the extraction buffer in an amount in the range from 100 to 300 mM, more preferably in a concentration equal to about 200 mM.

The extraction buffer also comprises a buffer solution at a pH higher than or equal to 8. Preferably said buffer solution is a buffer solution having a pH in the range 8≤pH≤9, more preferably the buffer is at pH 8.8.

Preferably said buffer solution is based on trishydroxymethylaminomethane hydrochloride (Tris-HCl), Trycine or glycine-glycine (Gly-Gly), being more preferably based on 20 mM Tris-HCl.

In an advantageous embodiment the extraction buffer comprises a buffer solution based on 20 mM Tris-HCl at pH 8.8 and dithiothreitol (DTT) as a reducing agent.

Said extraction buffer preferably also comprises a detergent. Said detergent is preferably at a concentration in the range from 0.5 to 4% by weight, preferably in a 2% concentration by weight. Said detergent is preferably chosen from the group consisting of sodium dodecylsulphate (SDS), sodium cholate, sodium deoxycholate, polyethylene glycol p-(1,1,3-tetramethylbutyl)-ether (Triton X100®), Polyoxyethylene (20) sorbitan monolaurate (Tween20®), octyl-β-glucopyranoside, tert-octylphenoxy poly(oxyethylene)ethane (Nonidet P40®); more preferably said detergent is SDS and is present in the extraction buffer at 2% by weight.

In a preferred embodiment, the extraction buffer comprises 20 mM Tris-HCl pH 8.8, 2% SDS, 200 mM DTT. This particular buffer composition has provided very efficient extraction results as will be evident from the experimental part that follows.

The extraction buffer optionally also comprises a further amido-sulphobetaine type detergent. It is preferably chosen from the group consisting of ASB-14, ASB-16, ASB-C7BzO, 3-[(3-Cholamidopropyl)dimethylammonio]-1-propanesulphonate (CHAPS), 3-[(3-Cholamidopropyl)dimethylammonio]-2-hydroxy-1-propanesulphonate (CHAPSO). Said further detergent is useful for extracting proteins of high lipid content and is preferably added after a first hot incubation in the aforedescribed extraction buffer.

Said further detergent is added to the extraction buffer preferably in the form of an aqueous solution in a concentration in the range from 5 to 15% by weight, and more preferably 10% in a ratio from 1:10 to 1:30 relative to the final extraction buffer, and more preferably 1:20 to obtain a final concentration in the extraction buffer equal to about 0.5%.

The process of the invention comprises the step iii) of incubating the FFPE sample in the extraction buffer at a temperature in the range from 55°C to 100°C. Preferably said incubation step iii) takes place in two substeps:
iii-first part) incubating the sample at a temperature in the range from 90 to 100°C for a time range of 15 to 25 minutes;
iii-second part) incubating the sample subjected to step iii-first part) at a temperature in the range from 55 to 85°C for a time range of 1.5-2.5 hours;
Following the incubation step the process comprises the step iv) of recovering the liquid portion of the incubation product of step iii) containing the extracted proteins.

Preferably the process of the invention consists of a step i) of deparaffinization of the FFPE sample. More preferably said step i) of deparaffinization is carried out with a solvent chosen from the group consisting of xylene, toluene, octane, octane/methanol and mixtures thereof. Said solvent is even more preferably xylene.

After the deparaffinization step i) the process of the invention can advantageously provide for a rehydration step on the FFPE sample in decreasing alcohol concentrations before subjecting it to the buffer addition step ii). Preferably said rehydration step is effected with ethanol at 100%, at 96% or at 70%.

After the hot incubation step iii - first part), preferably the extraction process can comprise a step of adding a further detergent of amido-sulphobetaine type.

In an extremely advantageous embodiment the process of the invention comprises the step of adding an extraction buffer comprising 20 mM Tris-HCl, pH 8.8, 2% SDS, 200 mM DTT.

The process of the invention, comprising all the preferred and advantageous steps, has provided very efficient protein extractions with extraction yields in the range from 100 to 200 µg per tissue slice.

Specifically, surprising extraction yield results were obtained by using microtome slices of FFPE tissue, of 10 µm in thickness, sliced from a 1 cm³ block of FFPE tissue.

Another aspect of the invention concerns a kit for extracting proteins from FFPE samples, as a combined product for simultaneous, separate or sequential use in the extraction process of the invention, comprising a) a buffer solution at a pH higher than or equal to 8; and b) a reducing agent at a concentration higher than or equal to 100 mM, in which said reducing agent is a compound chosen from the group consisting of dithiothreitol (DTT), dithioerythritol (DTE), tris(2-carboxyethyl)phosphine (TCEP), mercaptoethanol (MEA).

As the kit of the invention is a combined product for use in the process of the invention, it will advantageously include all the advantageous and preferred ingredients indicated above for the extraction buffer of the invention process. It will therefore preferably comprise at least one further detergent. In its advantageous form it will also comprise a further detergent of amido-sulphobetaine type.

Said kit more preferably comprises:
- a container C containing a mixture comprising: said buffer solution, said detergent and optionally said further detergent of amido-sulphobetaine type;
- a plurality of containers Dn each one containing a suitable amount of reducing agent to suspend in a suitable amount of said mixture upon use, so as to complete the preparation of a suitable amount of the extraction buffer of the invention;
- an instruction leaflet.

Optionally the kit can comprise an additional container containing an aqueous solution of said further detergent of amido-sulphobetaine structure, if not already present in recipient C.

Advantageously said kit comprises:
- a container C comprising 10 ml of a mixture comprising: said buffer solution, said detergent and optionally said further detergent of amido-sulphobetaine type;
- Ten containers D each containing 30 mg of reducing agent into which 1 ml of said mixture is added to prepare 1 ml of extraction buffer ready for use at point (ii) of the aforedescribed method.

Optionally if said further detergent of amido-sulphobetaine structure is not already present in said mixture, said kit will include an additional container containing 500 µl of a 10 % by weight aqueous solution of said further detergent of amido-sulphobetaine structure to be added into the process of the invention.

In the most preferred embodiment said mixture comprises 20 mM Tris-HCl at pH 8.8 and SDS at 2% and optionally ASB-14 at 0.5%, said reducing agent being preferably DTT.

Said addition detergent of amido-sulphobetaine structure, if not already present in said mixture, is preferably ASB-14 in aqueous solution form at 10% by weight.

The extraction buffer of the process of the invention once prepared from the kit of the invention can be stored at -20°C for a maximum of 5 days.

All the kit components can be stored at ambient temperature or stored in a refrigerated environment. In this latter case, should precipitates form, the components can be heated to ambient temperature or to 37°C prior to use.

Preferably said containers are Eppendorf type test tubes.

According to the invention the preferred quantification method after applying the extraction process of the invention is the Bradford method (Bradford, M., Anal Chem. 1976, 72, 248-254).

The aforesaid method of protein extraction from FFPE tissue is endowed with high efficiency and provides extracts with high yields and quality. The generated extracts are compatible with most of the classical proteomic methods and generate reliable and reproducible results. The extracts obtained with the process of the invention find application in analysis by SDS-PAGE, western immunoblotting and protein arrays. The proteins separated by SDS-PAGE and 2D-PAGE can then be advantageously analyzed by the techniques of mass spectrometry such as MALDI-TOF-MS and HPLC-nano-ESI-QTOF-MS/MS.

The present patent application presents comparative analyses of extracts of FFPE tissue obtained with the method of the present invention with respect to extracts from fresh-frozen tissue.

The invention will now be illustrated by way of some illustrative non-limiting examples of the process of the invention and by way of some examples of analysis of the extracted proteins. The examples that represent application of the analysis techniques have been undertaken in a comparative manner with fresh tissue samples.

For the analysis by protein arrays, the extracted proteins were brought to the desired concentration with extraction buffer then spotted onto the support, which was specifically nitrocellulose.

For the analysis by SDS-PAGE and western immunoblotting, the proteins were diluted to the desired concentration in Laemmli buffer, incubated at 95°C for 5 minutes and finally deposited in gel wells.

For the analysis by ELISA and two-dimensional electrophoresis, the proteins were precipitated with TCA/acetone, then resuspended in water in the first case and in the focusing solution in the second.

Following one- and two-dimensional electrophoresis, the separated proteins were cut out from the gel, hydrolyzed and analyzed by mass spectrometry according to conventional methods.

### EXPERIMENTAL PART

### Example 1: Preparation of the protein extracts

### Tissue samples

In order to optimize the extraction procedure and analysis procedure, tissue from sheep was used and obtained as follows. Immediately after butchering, portions of tissue of about 1 cm³ were cut from the femoral muscle and multiple replicas of the sample were immediately frozen at -80°C or fixed for 24 hours in 10% buffered formalin, then dehydrated in a series of increasing alcohol concentrations and finally embedded in paraffin. The samples from the human tissue bank were derived from biopsies of hyperplastic thyroid, normal liver, pulmonary carcinoid and carcinoma. Diagnosis was undertaken immunohistochemically during normal hospital procedures.

### Preparation of the protein extracts according to the process of the invention

The protein extracts were obtained from both fresh tissue (comparative preparation) and FFPE tissue (preparation of the invention).

The frozen-fresh tissue was reduced into fragments using a sterile scalpel and placed into 2 ml test tubes, immersed in the extraction buffer according to the present invention for a final tissue concentration of 5% w/v and subjected to 3 cycles of 3 minutes at 30 cycles per second in the mechanical homogenizer TissueLyser (Qiagen). The tissue homogenates were clarified for 10 minutes at 4°C at 12,000 x g, quantified using the Bradford method and stored at -80°C until required.

Five 10 micron microtome sections of FFPE tissue were placed into 2 ml test tubes, then deparaffinized by incubating at ambient temperature with xylene for 10 minutes. After each incubation, the tissue was centrifuged at 12,000 x g for 3 minutes. The incubation and centrifugation steps were repeated 3 times. The pellets thus obtained were directly treated for extraction or rehydrated in a series of decreasing alcohol concentrations (100%, 96%, 70% v/v). At this point the samples were subjected to the high temperature extraction process, according to the method of the present invention. The extraction buffer pH was 8.8, and reducing agent concentration (DTT) was 200 mM. The extraction under hot conditions was conducted using the following incubation method: 20 minutes at 100°C and 2 hours at 80°C with orbital agitation. The protein extracts were clarified by centrifugation, quantified using the Bradford method and stored at-80°C.

As a comparison, the protein extracts from fresh tissue were subjected to a high temperature heating protocol corresponding to that used for the protein extraction from FFPE tissue.

For the protein extract preparation the following detailed steps were followed:
- Deparaffinization of the FFPE samples by means of the following procedure:
   Five 10 micron slices cut from the paraffin-embedded block were resuspended and then placed in a 2 ml test tube with safety cap. 1 ml of xylene was placed in the test tube which was closed and vigorously agitated with a Vortex mixer for at least 10 seconds. The test tube was then incubated for 8 minutes at ambient temperature, centrifuged at 12,000 x g for 3 minutes and the supernatant removed. The step from incubation to supernatant removal was repeated twice more.
- Rehydration with ethanol at decreasing concentrations according to the following procedure:
   1 ml of 100% ethanol was dispensed into the test tube which was closed and vigorously agitated with a Vortex mixer for at least 10 seconds. This was followed by incubation for 8 minutes at ambient temperature, centrifugation at 12,000 x g for 5 minutes and supernatant removal. The steps of incubation, centrifugation and removal of supernatant were repeated twice more. 1 ml of 96% ethanol was then dispensed into the test tube which was closed and vigorously agitated with a Vortex mixer for at least 10 seconds. This was followed by incubation for 8 minutes at ambient temperature, centrifugation at 12,000 x g for 5 minutes and supernatant removal. The steps of incubation, centrifugation and supernatant removal were repeated twice more. 1 ml of 70% ethanol was then dispensed into the test tube which was closed and vigorously agitated with a Vortex mixer for at least 10 seconds. This was followed by incubation for 8 minutes at ambient temperature, centrifugation at 12,000 x g for 5 minutes and supernatant removal. The steps of incubation, centrifugation and supernatant removal were repeated twice more.
- Treatment with extraction buffer according to the following procedure:
   Added to the residual pellet were 150 microlitres of extraction buffer comprising the following:
      - 20 mM Tris-HCl at pH 8.8
      - 200 mM dithiothreitol (DTT)
      - 2% sodium dodecyl sulphate (SDS)

The mass was then vigorously agitated with a Vortex mixer for 10 seconds and incubated on ice for 10 minutes, then again vigorously agitated with a Vortex mixer and then incubated at 100°C for 20 minutes in a thermal block. Then amido-sulphobetaine-14 (ASB-14) was added at 10% in a 1:20 ratio to the mixture (final of 0.5%). The mass was then incubated at 80°C for 2 hours in a block agitated at 700 rpm.

### -Recovery of the liquid portion

The liquid portion containing the extracted proteins was then recovered by means of the following procedure:

The test tubes were placed on ice for 10 minutes, centrifuged at 14,000 g for 15 minutes; the supernatant was then recovered and placed in a new test tube.

The extracted proteins could be utilized or maintained at -20°C or at -70°C for long storage periods.

### Example 2: Preparation of the protein extracts

Five 10 micron microtome sections of FFPE tissue were placed into 2 ml test tubes, then deparaffinized by incubating at ambient temperature with octane for 10 minutes. After each incubation, the tissue was centrifuged at 12,000 x g for 3 minutes. The incubation and centrifugation steps were repeated 3 times. The pellets thus obtained were rehydrated in a series of decreasing alcohol concentrations (100%, 96%, 70% v/v). At this point the samples were subjected to the high temperature extraction process, according to the method of the present invention. The extraction buffer pH value was 8.8, in the absence of reducing agents (or in the presence of 50 mM DTT). The extraction under hot conditions was conducted using the following incubation method: 20 minutes at 100°C and 2 hours at 60°C in a thermostatically controlled block under static conditions. The protein extracts were clarified by centrifugation, quantified using the Bradford method and stored at -80°C.

As a comparison, the protein extracts from the fresh tissue were subjected to a heating protocol at high temperature corresponding to that used for the protein extraction from FFPE tissue.

### Example 3: Analysis of the protein extracts of example 1

### Electrophoresis on polyacrylamide gel (SDS-PAGE)

The protein extracts obtained from fresh and FFPE tissue from example 1 were subjected to two-dimensional electrophoresis according to Laemmli. The proteins were stained with Brilliant blue G 250 following the Westermeier protocol, and the image was digitized using an ImageScanner and processed using ImageQuant TL software.

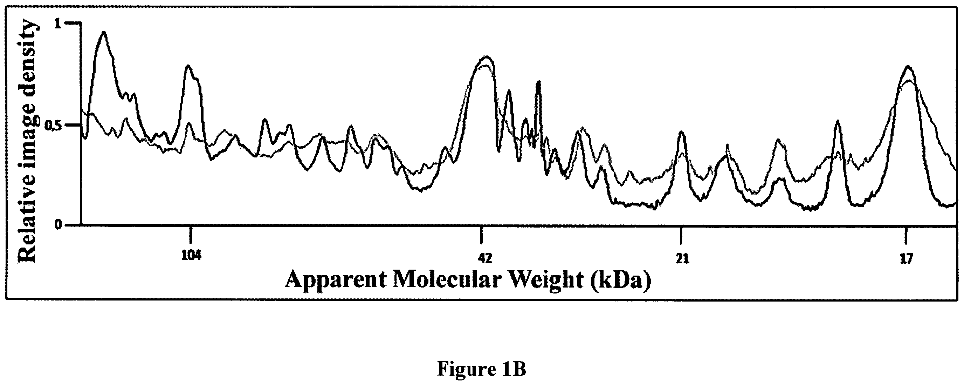

To demonstrate the applicability of the extractive method to samples deriving from FFPE human tissue banks and to verify its consistency and reproducibility, the extraction process typical of the present invention was applied to some representative human tissue: FFPE tissue sections from normal liver, hyperplastic thyroid, pulmonary carcinoid and carcinoma were subjected to the same extraction protocol with satisfying results (Figure 1).

Figure 1 shows the electrophoretic profile obtained by separating the proteins extracted from fresh and FFPE tissue. The protein profile corresponding to the FFPE extract appeared to be well resolved, the bands were clear and the relative abundance was comparable to that of fresh tissue. Also obtained was Figure 1 B which is a diagram showing a comparison between the relative image density profile of the FFPE extract and that of the fresh extract. As is evident there is a significant similarity between the areas of the peaks corresponding to the protein bands.

### Western immunoblotting

The proteins separated by electrophoresis were transferred onto a nitrocellulose membrane, blocked with a solution of 5% fat-free milk in PBS with 0.05% Tween20 for a time period from one to twelve hours then processed for the immunological detection.

With the aim of establishing the reactivity of the extraction products, all the obtained extracts were incubated with anti-actin antibodies as control. As shown in figure 1 (bottom), there can be seen a strong, clear and sharp band corresponding to actin at the level expected in all the electrophoretically separated protein extracts; this confirmed that the proteins had maintained their correct molecular weights and their immunoreactivity was successfully recovered.

The immunological reactivity of other proteins with higher or lower molecular weights than actin were then tested in the FFPE muscle extracts (Table 1).

**Table 1 - Proteins, antibodies (Ab) and dilutions used for immunological analysis (WB)**

| Protein (Ag) | MW (kDa) | Ab origin | Producer | WB Dilution | ELISA Dilution |
|---|---|---|---|---|---|
| Tropomyosin | 34 | Rabbit | Sigma-Aldrich | 1:5,000 | 1:1,000 |
| GAPDH | 36 | Rabbit | Sigma-Aldrich | 1:50,000 | 1:5,000 |
| Actin | 45 | Rabbit | Sigma-Aldrich | 1:50,000 | 1:5,000 |
| Desmin | 53 | Rabbit | Sigma-Aldrich | 1:5000 | 1:1,000 |
| Vinculin | 120 | Rabbit | Sigma-Aldrich | 1:5,000 | 1:1,000 |
| Myosin HC | 200 | Mouse | Millipore | 1:50,000 | 1:1,000 |

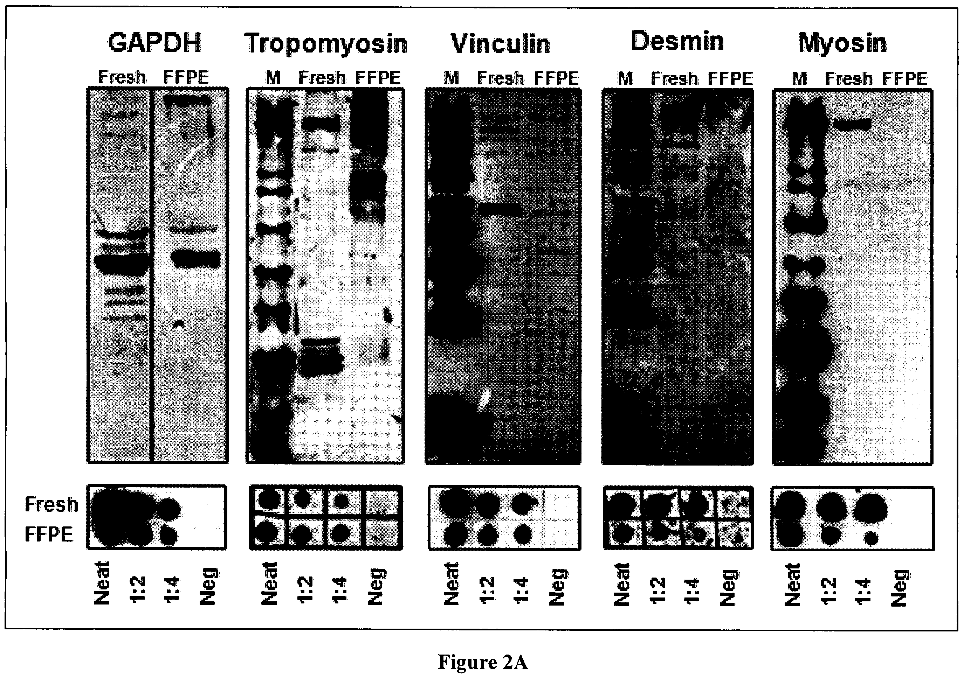

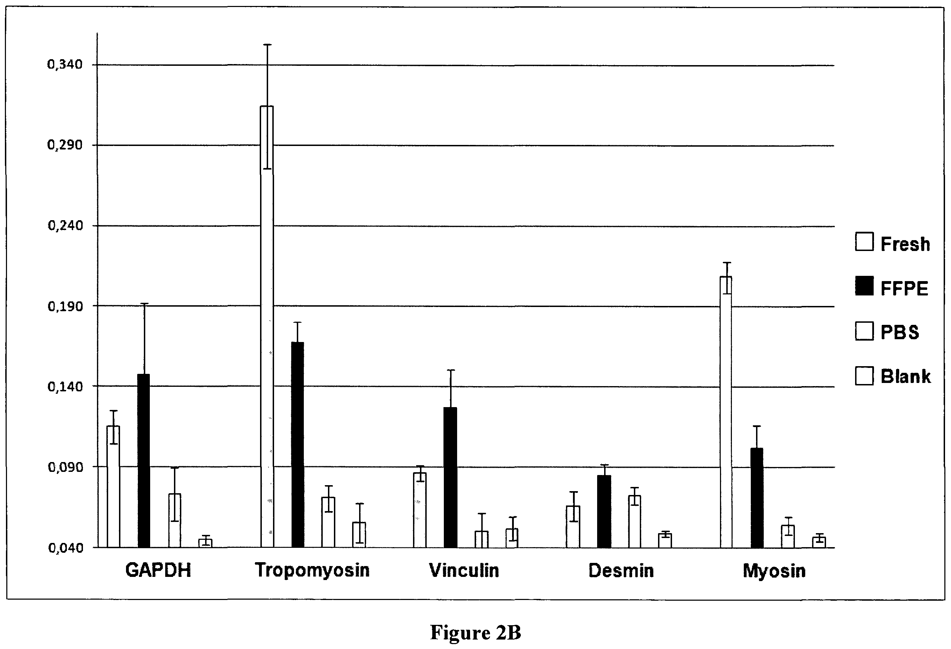

As shown in Figure 2A the following results were obtained:
GAPDH reactivity was found to be clear and consistent with the expected molecular weight (about 35 kDa); tropomyosin also presented a clear signal at the expected molecular weight, together with a cross-reactivity band in the upper part of the gel. Equally, desmin reactivity was found to be at the correct level, though with a weak signal. Vinculin, which has a higher molecular weight, presented a weak reactive band. On the other hand, myosin gave no signal with the antibody used in this analysis (Figure 2).

### Protein arrays

The protein extracts from fresh and FFPE tissue were spotted onto nitrocellulose sheets according to the following scheme:
1 microgram of total protein extract, 2 serial dilutions (1:2 and 1:4) of the extract in extraction buffer, and neat buffer as negative control.

The nitrocellulose sheet was allowed to dry out then blocked with a 5% fat-free milk solution in PBS with 0.05% Tween20 for a time period from one to twelve hours, followed by processing for immunological detection. Antigenic reactivity was detected by using anti-mouse or anti-rabbit secondary antibodies conjugated to peroxidise, through the addition of a chemiluminescent peroxidase substrate.

The FFPE extracts obtained with the method of the present invention were tested using the array technique. All the tested antibodies produced a strong, reproducible and quantitative signal in the FFPE extracts, including vinculin and myosin which had not given very good results with western blotting. Tropomyosin on the other hand showed an interesting result; in this respect a difference in signal intensity could be noted between the fresh and FFPE extracts: this behaviour can be related to the cross-reactivity band already noted in the blot with the same antibody.

### ELISA

The ELISA experiments were carried out according to the following protocol. The total protein extracts from fresh and FFPE tissue obtained by the method of the present invention were precipitated with trichloroacetic acid/acetone according to standard procedures, then quantified and resuspended in PBS at a concentration of 100 micrograms/ml. 100 microlitres of sample were dispensed into wells of a maxisorp plate and incubated at ambient temperature until dry. At this point the wells were washed and blocked with a solution of PBS-T with 5% albumin for one hour. After washing with PBS-T, the primary antibody was added to each well, incubated for two hours at ambient temperature and washed five times with PBS-T. The appropriate secondary antibody was then added and the wells were incubated for one hour at 37°C and washed as above. After incubation, the reactivity controls (water as such, neat PBS, secondary antibody as such) were run in parallel with each experiment. A chromogenic substrate was then added to all the wells and absorbance at 650 nm was read every 15 minutes for one hour. The assays were replicated in triplicate and the mean and standard deviation were calculated and represented graphically with the aid of Microsoft Excel. Antibodies and solutions are schematically represented in table 1.

The ELISA technique was successfully applied to all the tested antibodies as shown in Figure 2B. In all cases it can be seen that higher absorbance values are obtained for the samples compared to the controls, which consisted of PBS and water. Furthermore, the values obtained for the FFPE extracts were generally close or comparable to those obtained with fresh tissue.

### In Situ hydrolysis of protein bands obtained by Electrophoresis on polyacrylamide gel (SDS-PAGE)

After staining with colloidal Coomassie, the 21 visible bands were cut out of the runs corresponding to the fresh and FFPE extracts; this was subsequently followed by decolorization by means of repeated sequential washing with 50 mM ammonium bicarbonate at pH 8.0 and acetonitrile. The samples were then reduced by incubating at 56°C for 45 minutes in a solution of 10 mM DTT in 50 mM ammonium bicarbonate and carbamidomethylated by incubating for 30 minutes in darkness at ambient temperature in a solution of 55 mM iodoacetamide in 50 mM ammonium bicarbonate. The alkylated samples were then digested overnight with trypsin at 37°C, using 100 ng of enzyme for each band.

### Tandem mass spectrometry analysis

The LC-MS/MS analyses were conducted on a hybrid Q-TOF mass spectrometer equipped with a nano-lock Z-spray source and coupled on line to a capillary chromatographic system, CapLC. After injection, the peptide mixture was concentrated and washed at 20 microlitres/minute in a reverse-phase precolumn using 0.2% formic acid as eluent. The sample was then fractionated in a reverse-phase C₁₈ capillary column at a flow rate of 250 nanolitres/minute, utilizing a linear gradient of eluent B in eluent A. The mass spectrometry setting was in data-dependent MS/MS mode in which one complete analysis spectrum was followed by a tandem mass spectrum. The peptide ions were selected from the most intense peaks of the preceding analysis. A suitable collision energy was applied relative to the mass and the charge of the precursor ion. ProteinLynx software was used to analyze the spectra and to generate a list of peaks which was input into the Mascot software for protein identification. Gene classifications were assigned with the aid of GoMiner software and graphs were produced with Microsoft Excel.

SDS-PAGE separation was used as a prefractionation step for an *in-situ* approach to hydrolysis which would enable a tandem mass analysis of the FFPE tissue extracts with a tandem mass spectrometer. Table 2 gives the identity of all the proteins identified from fresh (A) and FFPE (B) skeletal muscle tissue of sheep.

For each protein the number of matched peaks, sequence coverage, Mowse score, nominal mass, calculated isoelectric point value and Swiss-Prot identification number are given. As shown in Figure 3, 85 and 66 proteins in total were identified from fresh and FFPE tissue respectively. In total 64% (corresponding to percentage identity derived from superimposing the absolute number shown in the graph) of the proteins identified from fresh tissue are also identified in FFPE tissue, while 85% of the proteins identified in FFPE are also identified in fresh tissue. Within the 50 most represented proteins, 80% are identified in both fresh and FFPE tissue.

The peptides obtained from trypsin digestion of the proteins extracted from FFPE tissue generated fragmentation mass spectra comparable with those obtained from fresh extracts.

Figure 4 shows six representative spectra obtained by carbonic anhydrase 3, alpha actinin 2 and NADH dehydrogenase, respectively, in fresh and FFPE tissue. Fixation with formalin appeared not to have damaging effects on the quality of the mass spectra obtained from fresh and FFPE tissue.

To compare the quality and reproducibility of the extraction, all the proteins identified from fresh and FFPE tissue were analyzed for molecular function and cell localisation. Figure 5 shows the gene classification for both extracts. Although a few small differences can be seen, the distribution according to class and relative abundance are mostly preserved; thus, fixation with formalin prior to extraction appeared not to introduce significant errors regarding particular functions or cell localisations, which demonstrates the effectiveness of the extraction process of the present invention.

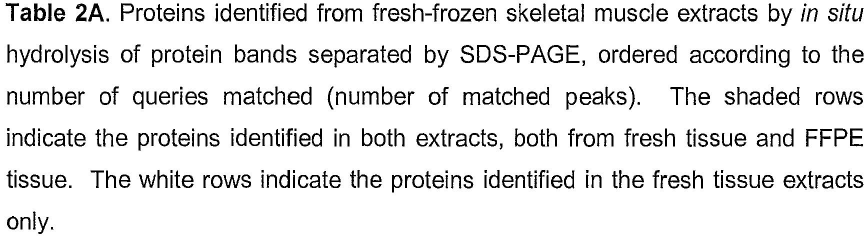

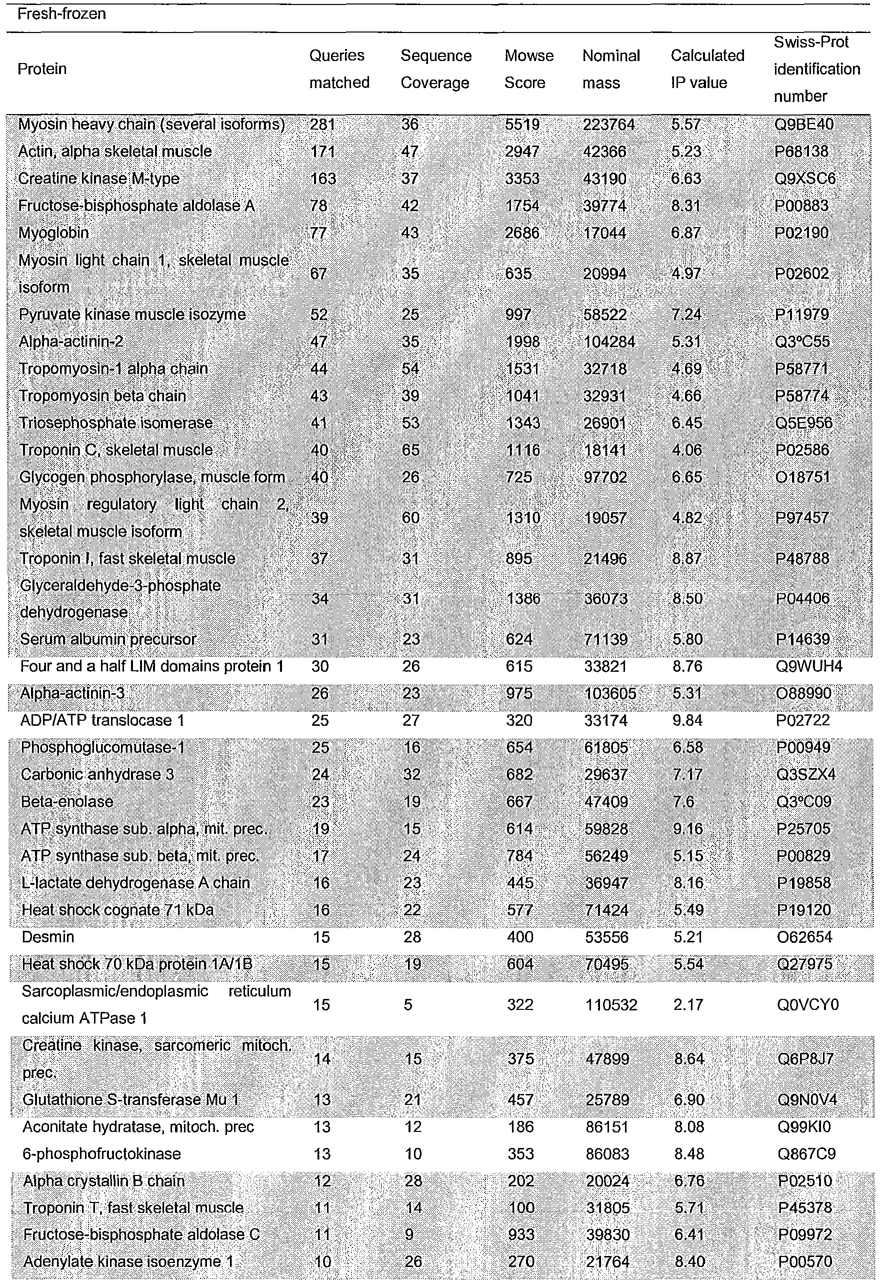

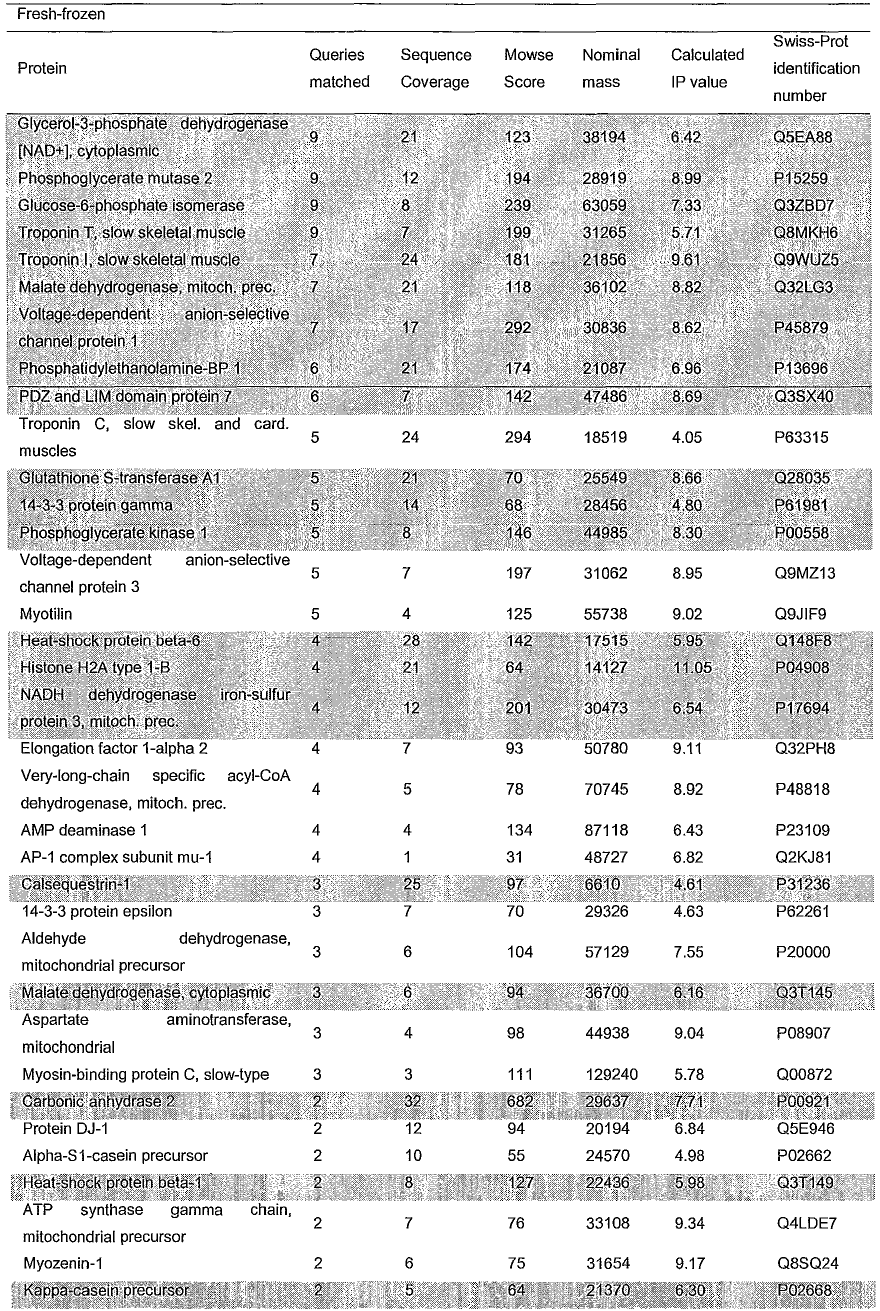

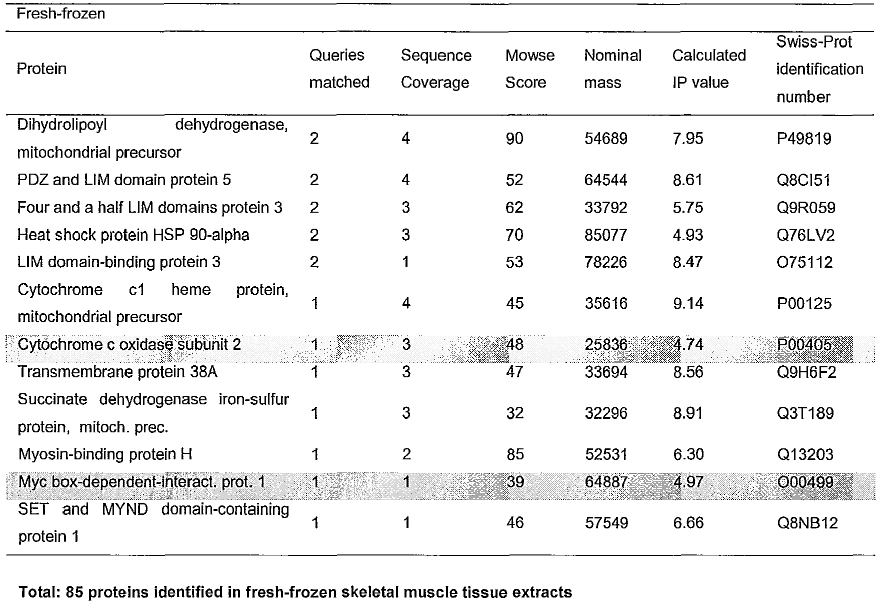

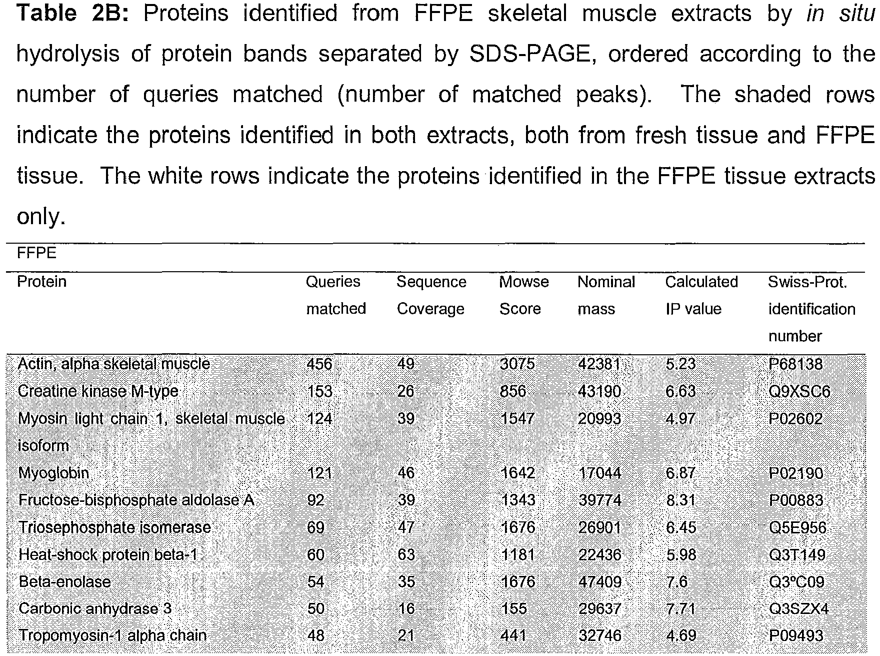

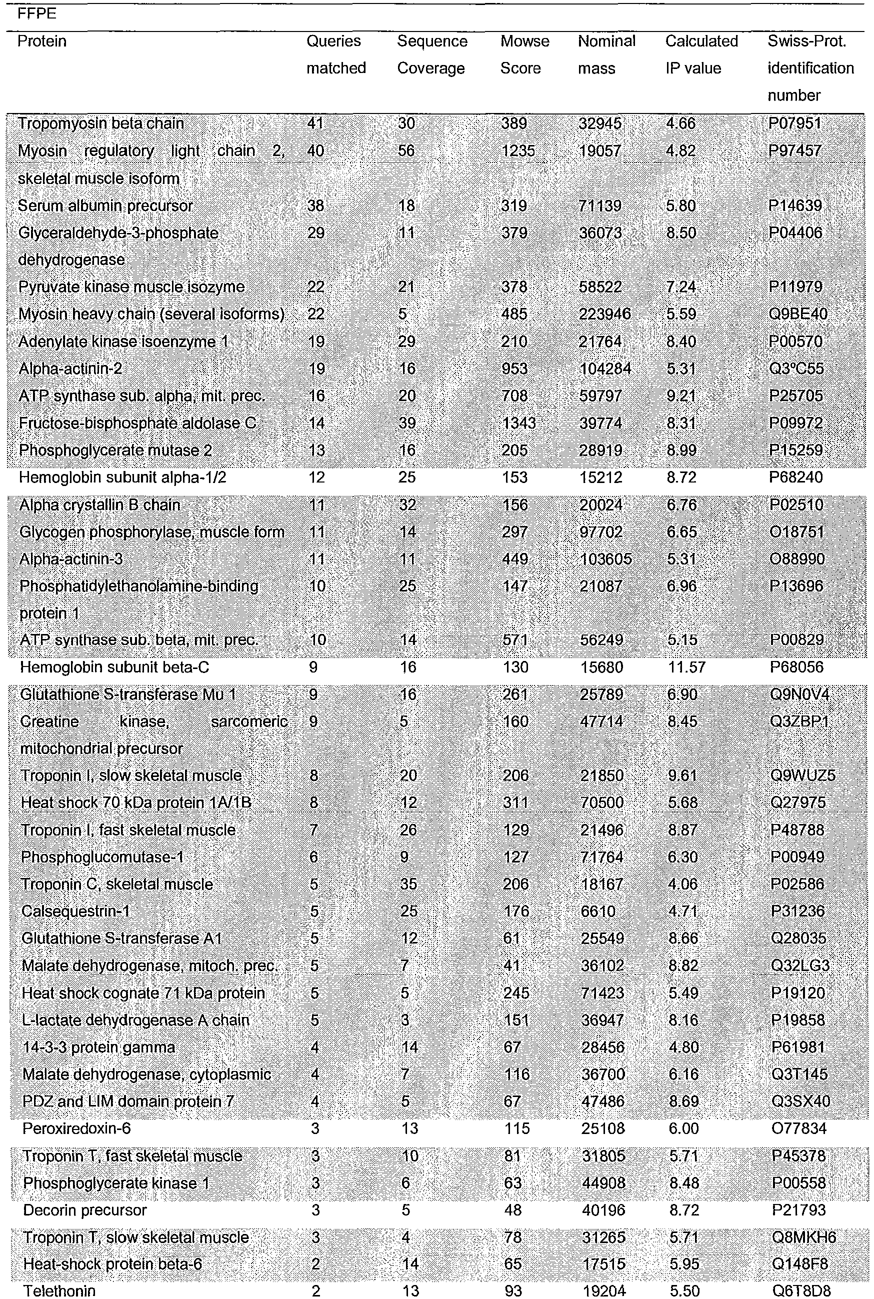

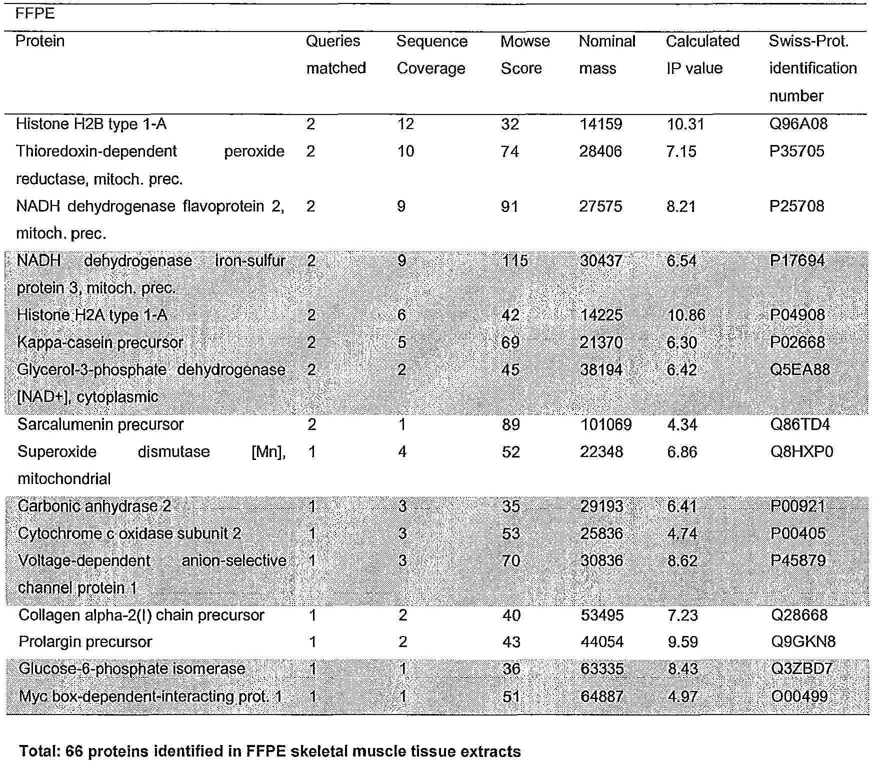

As is evident from Table 2B, 66 proteins were identified in FFPE muscle extracts compared to 85 in the fresh samples, demonstrating the effectiveness of the process of the invention in providing protein extract samples which can be analyzed with the most common and advanced proteomic techniques.

### Comparative example

The process of the invention was compared with a process carried out using the commercial QProteome FFPE Tissue kit. The kit is based on the process of patent application WO 2006/122898 A1. In Table 3 the tested composition of the buffers of patent application WO2006/122898 A1 and those of the present invention are listed.

**Tabella 3.**

| Components | Extraction buffer (EB1) described in the prior art example (WO 2006/122898 A1) | Extraction buffer used in the current invention | | Alternative extraction buffer (EB2) described in the prior art example (WO 2006/122898 A1) | Alternative extraction buffer used in the current invention |
|---|---|---|---|---|---|
| Chemical conditions | | | | | |
| pH | 6.8 | 8.8 | | 6.8 | 8.8 |

| Salts and buffers | | | | | |
|---|---|---|---|---|---|
| Bicine | 12.5 mM | - | | - | - |
| Sodium chloride | 75 mM | - | | - | - |
| Tris | 6.25 mM | 20 mM | | 90 mM | 20 mM |

| Detergents | | | | | |
|---|---|---|---|---|---|
| SDS | 1% | 2% | | 2% | 2% |
| ASB-14 | - | - | | - | 0.5% |
| T-PER^{®} detergent from Pierce | 0.5% | - | | - | - |

| Other components | | | | | |
|---|---|---|---|---|---|
| Glycerin | 9% | - | | 20% | - |

| Reducing agents | | | | | |
|---|---|---|---|---|---|
| Dithiothreitol | - | 100mM | | 1mM | 100mM |
| 2-mercaptoethanol | 5,6% | - | | - | - |

The process of the invention always produced better results in terms of amount and quality of the extracted proteins after analysis by SDS-PAGE. Protein extraction from three replicates of 5 FFPE skeletal muscle tissue slices was about 48.6±0.5 SD µg/ml in the case of the process of the present invention, and about 8.2±1.1 SD µg/mt in the case of the process of patent application WO2006/122898 A1. Figure 6 illustrates the result obtained after the following: 1) extraction from fresh skeletal muscle tissue; 2) extraction from FFPE ovine skeletal muscle tissue using the method described herein; 3) extraction from FFPE ovine skeletal muscle tissue using the method described in patent WO2006/122898 A1. The result obtained with the process of the invention is better both in terms of amount of protein extracted and the number of observable protein bands in the sample. In the image shown, loading of the sample in lane 3 (process of the prior art) was increased with the aim of being able to compare the protein signals in the two FFPE extracts.

The yield, quality and reproducibility of the extraction were also evaluated by means of western blotting with anti-actin antibodies. Replicate experiments were designed, following which the efficiency of protein extraction was always found to be better with the process of the invention than the method described in patent WO2006/122898 A1. Furthermore, in some of the extraction replicates the prior art method resulted in no signal corresponding to actin being detected; conversely the process of the invention reproducibly generated a positive signal in all the extraction replicates.

Figure 7 illustrates a result representative of that obtained after electrophoresis and western immunoblotting with anti-actin antibodies. 1) actin signal obtained following protein extraction from fresh skeletal muscle tissue; 2) actin signal obtained following protein extraction from five 10µm slices of FFPE skeletal muscle using the present process; 3) actin signal obtained following protein extraction from five 10µm slices of FFPE skeletal muscle using the method of patent application WO2006/122898 A1. In case 1), 1 microlitre of extract was loaded onto each lane; in cases 2 and 3, 5 microlitres of extract were loaded onto each lane.

The extract obtained from fresh and FFPE skeletal muscle with the process of the invention was also analyzed by two-dimensional electrophoresis (Figure 8), two-dimensional western immunoblotting (Figure 9) and mass spectrometry (Figure 10), to produce never before published results. The extract using the method of patent application WO2006/122898 A1 did not produce acceptable results. The inadequacy of the method of patent application WO2006/122898 A1 in generating protein extracts of suitable quality for analysis with the same applications is also clearly demonstrated in the recent scientific literature: "Proteomic analysis of RCL2 paraffin-embedded tissue. J. Cell. Mol. Med. Vol. 12, No. 5B, 2008, pp 2027-2036", Figures 1(D) and 2(D). The authors of this work declare that: "Using the QProteome FFPE kit" i.e. the method of patent application WO2006/122898 A1, "we observed a completely degraded protein profile on 1-DE and 2-DE gels. Nuclear and cytoplasmic proteins could not be detected by western blot".

## Claims

1. A process for extracting proteins from FFPE (Formalin-Fixed Paraffin-Embedded) tissue samples comprising the following steps:
i) deparaffinizing the FFPE sample,
ii) adding to the deparaffinized FFPE sample an extraction buffer comprising:
A) a buffer solution at pH higher than or equal to 8; and
B) a reducing agent in a concentration higher than or equal to 100mM;
iii) incubating the FFPE sample in the extraction buffer at a temperature in the range from 55 °C to 100°C,
wherein said reducing agent is a compound selected from the group consisting of dithiothreitol (DTT), dithioerythritol (DTE), tris(2-carboxyethyl)phosphine (TCEP), mercaptoethanol (MEA).

2. The process according to Claim 1, wherein said reducing agent is the compound dithiothreitol (DTT).

3. The process according to Claim 1 or 2, wherein said reducing agent is present in the extraction buffer in an amount in the range from 100 to 300 mM, preferably in an amount equal to about 200mM.

4. The process according to any one of Claims 1-3, wherein said buffer solution is a buffer solution having a pH in the range 8 ≤pH≤ 9, more preferably the buffer is at pH 8.8.

5. The process according to any one of Claims 1-4, wherein said buffer solution is selected from the group consisting of a buffer solution based on trishydroxymethylaminomethane hydrochloride (Tris-HCl), buffer solution based on Tricine and buffer solution based on glycine-glycine (Gly-Gly).

6. The process according to any one of Claims 1-5, wherein said extraction buffer also comprises a detergent.

7. The process according to Claim 6, wherein said detergent is in a concentration in the range from 0.5 to 4 % by weight, preferably in a concentration of 2% by weight, and is selected from the group consisting of sodium dodecyl sulphate (SDS), sodium cholate, sodium deoxycholate, polyethylene-glycol p-(1,1,3-tetramethylbutyl)-ether, polyoxyethylene (20) sorbitan monolaurate, octyl-β-glucopyranoside, tert-octylphenoxy poly(oxyethylene)ethane, and mixtures thereof.

8. The process according to any one of claims 1-7, wherein the extraction buffer comprises a further detergent of amido-sulphobetaine type.

9. The process according to Claim 8, wherein said further detergent is added to the extraction buffer in the form of an aqueous solution in a concentration in the range from 5 to 15% by weight, more preferably 10%.

10. The process according to any one of Claims 1-9, wherein the step iii) is carried out in two substeps:
iii- first part) incubating the sample at a temperature in the range from 90 to 100°C for a time range of 15-25 min;
iii- second part) incubating the sample subjected to the step iii-first part) at a temperature in the range from 55 to 85°C for a time range of 1.5-2.5 hours.

11. The process according to any one of Claims 1-10, wherein following the incubation step iii), the process provides for a step iv) of recovering the liquid portion of the incubation product of step iii) containing the extracted proteins.

12. The process according to any one of Claims 1-11, wherein after the step i) of deparaffinization, said process comprises a step of rehydrating the FFPE sample in decreasing alcohol concentrations before subjecting it to the extraction buffer addition step ii).

13. Use of a kit for protein extraction process according to any one of Claims 1-12, said kit as a combined product for simultaneous, separate or sequential use, said kit comprising a) a buffer solution at pH higher than or equal to 8; and b) a reducing agent in a concentration higher than or equal to 100mM, wherein said reducing agent is a compound selected from the group consisting of dithiothreitol (DTT), dithioerythritol (DTE), tris(2-carboxyethyl) phosphine (TCEP), mercaptoethanol (MEA).

14. Use according to Claim 13, wherein the kit comprises:
- a container C containing a mixture comprising the buffer solution and the detergent;
- a plurality of containers Dn each containing a suitable amount of reducing agent to be suspended in a suitable amount of said mixture upon use, so as to complete the preparation of a suitable amount of the extraction buffer of the invention;
- an instruction leaflet.

15. Use according to Claim 14, wherein in the kit a further detergent of amido-sulphobetaine type is comprised in container C or in a further container containing an aqueous solution.

## Patentansprüche

1. Verfahren zur Extraktion von Proteinen aus FFPE (Formalin-Fixed Paraffin-Embedded) Gewebeproben, aufweisend die folgenden Schritte:
i) Entparaffinierung der FFPE Probe
ii) Zugabe eines Extraktionspuffers zur entparaffinierten FFPE Probe, aufweisend:
A) eine Pufferlösung mit einem pH-Wert größer gleich 8 und
B) ein Reduktionsmittel in einer Konzentration größer gleich 100 mM;
iii) Inkubieren der FFPE-Probe in dem Extraktionspuffer bei einer Temperatur im Bereich zwischen 55°C und 100°C,
wobei das Reduktionsmittel eine Mischung ist, die ausgewählt wird aus der Gruppe von Dithiothreitol (DTT), Dithioerythritol (DTE), Tri(2-Carboxyethyl)Phosphin (TCEP) und Mercaptoethanol (MEA).

2. Verfahren gemäß Anspruch 1, wobei das Reduktionsmittel die Verbindung Dithiothreitol (DTT) ist.

3. Verfahren gemäß Anspruch 1 oder 2, wobei das Reduktionsmittel in dem Extraktionspuffer in einer Menge im Bereich zwischen 100 bis 300 mM vorliegt, vorzugsweise in einer Menge von etwa 200 mM.

4. Verfahren gemäß einem der Ansprüche 1 bis 3, wobei die Pufferlösung einen pH-Wert von 8 bis 9 aufweist, vorzugsweise liegt der pH-Wert des Puffers bei 8,8.

5. Verfahren gemäß einem der Ansprüche 1 bis 4, wobei die Pufferlösung ausgewählt wird aus der Gruppe von Pufferlösungen bestehend aus einer Pufferlösung basierend auf Trihydroxymethylaminomethan-Hydrochlorid (Tri-HCl), einer Pufferlösung basierend auf Tricin und einer Pufferlösung basierend auf Glycin-Glycin (Gly-Gly).

6. Verfahren gemäß einem der Ansprüche 1 bis 5, wobei der Extraktionspuffer zudem ein Detergens aufweist.

7. Verfahren gemäß Anspruch 6, wobei das Detergens in einer Konzentration von 0,5 bis 4 Gew.-% vorliegt, vorzugsweise in einer Konzentration von 2 Gew.-%, und ausgewählt wird aus der Gruppe bestehend aus Natriumdodecylsulfat (SDS), Natriumcholat, Natriumdeoxycholat, Polyethylen-Glykol p-(1,1,3-Tetramethylbutyl)-Ether, Polyoxyethylen-(20)-Sorbitanmonolaurat, Oktyl-β-Glukopyrosid, tert-Octylphenoxy-Poly(oxyethylen)ethan und Mischungen daraus.

8. Verfahren gemäß einem der Ansprüche 1 bis 7, wobei der Extraktionspuffer ein weiteres Detergens des Amido-Sulfobetain-Typs aufweist.

9. Verfahren gemäß Anspruch 8, wobei das weitere Detergens in Form einer wässrigen Lösung in einer Konzentration von 5 bis 15 Gew.-%, vorzugsweise 10%, dem Extraktionspuffer hinzugefügt wird.

10. Verfahren gemäß einem der Ansprüche 1 bis 9, wobei Schritt iii) in zwei Unterschritten durchgeführt wird:
iii- erster Teil) Inkubieren der Probe bei einer Temperatur im Bereich von 90°C bis 100°C für eine Dauer von 15 bis 25 min.;
iii- zweiter Teil) Inkubieren der Probe aus iii- erster Teil) bei einer Temperatur im Bereich von 55°C bis 85°C für eine Dauer von 1,5 bis 2,5 Stunden.

11. Verfahren gemäß einem der Ansprüche 1 bis 10, wobei das Verfahren nach Schritt iii) einen Schritt iv) vorsieht, in dem der flüssige Teil des Inkubationsprodukts aus Schritt iii) zurückgewonnen wird, der die extrahierten Proteine enthält.

12. Verfahren gemäß einem der Ansprüche 1 bis 11, wobei nach dem Schritt i), der Entparaffinierung, das Verfahren einen Schritt der Rehydrierung der FFPE-Probe in abnehmenden Alkoholkonzentrationen vorsieht, bevor es Schritt ii), der Zugabe des Extraktionspuffers, unterzogen wird.

13. Verwendung eines Kit für ein Verfahren zur Proteinextraktion gemäß einem der Ansprüche 1 bis 12, wobei das Kit ein Kombinationsprodukt zur simultanen, separaten oder fortlaufenden Verwendung ist, das Kit aufweisend
a) eine Pufferlösung mit einem pH-Wert größer gleich 8; und
b) ein Reduktionsmittel in einer Konzentration größer gleich 100 mM;
wobei das Reduktionsmittel eine Verbindung ist, die ausgewählt wird aus der Gruppe von Dithiothreitol (DTT), Dithioerythritol (DTE), Tri(2-Carboxyethyl)Phosphin (TCEP) und Mercaptoethanol (MEA).

14. Verwendung gemäß Anspruch 13, wobei das Kit aufweist:
- einen Behälter C, der eine Mischung aus der Pufferlösung und dem Detergens beinhaltet;
- eine Vielzahl von Behältern Dn, die jeweils eine geeignete Menge Reduktionsmittel enthalten, um bei Verwendung in einer geeigneten Menge der Mischung aufgelöst zu werden, so dass die Zubereitung einer geeigneten Menge des Extraktionspuffers der Erfindung fertiggestellt werden kann;
- einen Beipackzettel.

15. Verwendung gemäß Anspruch 14, wobei von dem Kit ein weiteres Detergens des Amido-Sulfobetain-Typs in einem Behälter C oder in einem weiteren Behälter, der eine wässrige Lösung enthält, umfasst ist.

## Revendications

1. Procédé d'extraction de protéines à partir d'échantillons tissulaires FFPE (fixés au formol et enrobés de paraffine) comprenant les étapes suivantes :
i) le déparaffinage de l'échantillon FFPE,
ii) l'ajout à l'échantillon FFPE déparaffiné d'un tampon d'extraction comprenant :
A) une solution tampon à un pH supérieur ou égal à 8 ; et
B) un agent réducteur à une concentration supérieure ou égale à 100 mM ;
iii) la mise en incubation de l'échantillon FFPE dans le tampon d'extraction à une température située dans la plage allant de 55 °C à 100 °C,
dans lequel ledit agent réducteur est un composé choisi dans le groupe constitué par le dithiothréitol (DTT), le dithioérythritol (DTE), la tris(2-carboxy-éthyl)phosphine (TCEP), le mercaptoéthanol (MEA).

2. Procédé selon la revendication 1, dans lequel ledit agent réducteur est le composé dithiothréitol (DTT).

3. Procédé selon la revendication 1 ou 2, dans lequel ledit agent réducteur est présent dans le tampon d'extraction en une quantité située dans la plage allant de 100 à 300 mM, de préférence en une quantité égale à environ 200 mM.

4. Procédé selon l'une quelconque des revendications 1 à 3, dans lequel ladite solution tampon est une solution tampon ayant un pH situé dans la plage 8 ≤ pH ≤ 9, de préférence le tampon a un pH de 8,8.

5. Procédé selon l'une quelconque des revendications 1 à 4, dans lequel ladite solution tampon est choisie dans le groupe constitué par une solution tampon à base de chlorhydrate de trishydroxy-méthylaminométhane (Tris-HCl), une solution tampon à base de tricine et une solution tampon à base de glycine-glycine (Gly-Gly).

6. Procédé selon l'une quelconque des revendications 1 à 5, dans lequel ledit tampon d'extraction comprend également un détergent.

7. Procédé selon la revendication 6, dans lequel ledit détergent est à une concentration située dans la plage allant de 0,5 à 4 % en poids, de préférence à une concentration de 2 % en poids, et est choisi dans le groupe constitué par le dodécylsulfate de sodium (SDS), le cholate de sodium, le désoxycholate de sodium, le polyéthylèneglycol p-(1,1,3-tétraméthylbutyl)-éther, le monolaurate de sorbitane polyoxyéthyléné (20), l'octyl-β-glucopyranoside, le tert-octylphénoxy poly(oxy-éthylène)éthane, et leurs mélanges.

8. Procédé selon l'une quelconque des revendications 1 à 7, dans lequel le tampon d'extraction comprend un détergent supplémentaire de type amido-sulfobétaïne.

9. Procédé selon la revendication 8, dans lequel ledit détergent supplémentaire est ajouté au tampon d'extraction sous la forme d'une solution aqueuse à une concentration située dans la plage allant de 5 à 15 % en poids, de préférence de 10 %.

10. Procédé selon l'une quelconque des revendications 1 à 9, dans lequel l'étape iii) est réalisée en deux sous-étapes :
iii- première partie) la mise en incubation de l'échantillon à une température située dans la plage allant de 90 à 100 °C pendant une durée de 15 à 25 minutes ;
iii- seconde partie) la mise en incubation de l'échantillon soumis à l'étape iii-première partie) à une température située dans la plage allant de 55 à 85 °C pendant une durée de 1,5 à 2,5 heures.

11. Procédé selon l'une quelconque des revendications 1 à 10, dans lequel, après l'étape iii) d'incubation, le procédé comprend une étape iv) de récupération de la partie liquide du produit d'incubation de l'étape iii) contenant les protéines extraites.

12. Procédé selon l'une quelconque des revendications 1 à 11, dans lequel, après l'étape i) de déparaffinage, ledit procédé comprend une étape de réhydratation de l'échantillon FFPE dans des concentrations décroissantes d'alcool avant de le soumettre à l'étape ii) d'ajout du tampon d'extraction.

13. Utilisation d'un kit pour un procédé d'extraction de protéines selon l'une quelconque des revendications 1 à 12, ledit kit en tant que produit combiné pour une utilisation simultanée, séparée ou séquentielle, ledit kit comprenant a) une solution tampon à un pH supérieur ou égal à 8 ; et b) un agent réducteur à une concentration supérieure ou égale à 100 mM, dans laquelle ledit agent réducteur est un composé choisi dans le groupe constitué par le dithiothréitol (DTT), le dithioérythritol (DTE), la tris(2-carboxyéthyl)-phosphine (TCEP), le mercaptoéthanol (MEA).

14. Utilisation selon la revendication 13, dans laquelle le kit comprend :
- un récipient C contenant un mélange comprenant la solution tampon et le détergent ;
- une pluralité de récipients Dn contenant chacun une quantité appropriée d'agent réducteur à mettre en suspension dans une quantité appropriée dudit mélange lors de l'utilisation, afin de terminer la préparation d'une quantité appropriée du tampon d'extraction de l'invention ;
- une notice d'instructions.

15. Utilisation selon la revendication 14, dans laquelle, dans le kit, un détergent supplémentaire de type amido-sulfobétaïne est compris dans le récipient C ou dans un récipient supplémentaire contenant une solution aqueuse.
